# FASCICULE DE BREVET EUROPEEN

(11) **EP 3 143 341 B1**
(45) Date de publication et mention de la délivrance du brevet: **19.08.2020**
(21) Numéro de dépôt: 15732025.0
(22) Date de dépôt: 12.05.2015
(51) Int. Cl.: F24F 6/00, F04D 25/08, F04D 27/00, F24F 7/007, F24F 11/00, F24F 13/078, F21V 33/00, G08C 17/02

(54) **PROCEDE DE COMMANDE D'UN VENTILATEUR**
VERFAHREN ZUR STEUERUNG EINES LÜFTERS
METHOD FOR CONTROLLING A FAN

(30) Priorité: 14.05.2014 FR 1454306
(43) Date de publication de la demande: 22.03.2017
(73) Titulaire: SEB S.A., 69130 Ecully (FR)
(72) Inventeur: CUBIZOLLES, Serge, F-38780 Oytier Saint Oblas (FR); ABERBACHE, Belkacem, F-74150 Rumilly (FR); LAVILLAT, Olivier, F-74600 Quintal (FR)
(74) Mandataire: Soares, Luis Filipe
(86) Numéro de dépôt international: PCT/FR2015/051243
(87) Numéro de publication internationale: WO 2015/173509

(56) Documents cités:
- US-A- 4 711 161
- US-A- 4 719 446
- US-A- 4 818 920
- US-A- 5 189 412
- US-A- 5 528 229
- US-A- 5 791 763
- US-A1- 2012 033 419

## Description

L'invention concerne de manière générale un système de ventilation qui agit sur l'air d'une pièce d'habitation.

Plus particulièrement l'invention concerne un système de ventilation qui fonctionne selon des programmes préenregistrés.

Dans un autre domaine on connait également le document US2012033419 qui divulgue un appareil d'éclairage optique à semi-conducteurs pour générer une lumière d'ambiance et qui intègre une ventilation des composants.

On connait des appareils de ventilation qui sont équipés d'une unité de contrôle qui permet de gérer des moyens de ventilation intégrés à l'appareil de ventilation. Par exemple l'unité de contrôle permet de faire fonctionner les moyens de ventilation à plusieurs vitesses de fonctionnement. Dans un autre exemple l'unité de contrôle permet de faire fonctionner les moyens de ventilation dans un certain sens de rotation ou dans un autre. Certains appareils de ventilation comprennent des moyens d'éclairage en parallèle des moyens de ventilation qui peuvent également être gérés par l'unité de contrôle.

Ainsi le document US5528229 décrit un appareil de ventilation qui est adapté à être fixé à un plafond d'une pièce d'habitation. Il comprend des moyens de ventilation, des moyens d'éclairage et un capteur de température. L'appareil de ventilation comprend une unité de contrôle qui permet de gérer l'ensemble et cette unité de contrôle comprend un ou plusieurs programmes de fonctionnement. Un premier programme de fonctionnement permet d'activer les moyens de ventilation à différentes vitesses ou selon un sens de rotation particulier. De plus dans ce programme de fonctionnement, la température d'une pièce d'habitation est mesurée par le capteur de température et la vitesse des moyens de ventilation est adaptée afin de refroidir ou d'homogénéiser la température de la pièce d'habitation. Ceci permet donc de réguler la température de la pièce d'habitation et cette régulation se fait de manière autonome en fonction du programme de fonctionnement sélectionné qui comprend une consigne de température préenregistrée. L'unité de contrôle comprend également un second programme de fonctionnement du moyen d'éclairage. Ce second programme de fonctionnement permet de gérer l'allumage du moyen d'éclairage. Cependant les opérations d'éclairage sont des opérations de base sur le luminaire comme par exemple l'allumage, l'extinction ou le réglage de l'intensité du luminaire selon un planning défini et selon des valeurs d'intensités discrétisées. Dans ce document, les moyens de ventilation et d'éclairage fonctionnent de manière indépendante.

Un des inconvénients d'un tel appareil de ventilation réside dans le fait que l'unité de contrôle doit posséder au moins deux systèmes de contrôle différents afin de gérer distinctement l'allumage et la ventilation selon les programmes de fonctionnement sélectionnés. Ceci a alors un impact sur le coût du système. Un des buts de la présente invention est de proposer un dispositif qui soit donc moins coûteux en simplifiant le schéma de contrôle. Un autre inconvénient est que les fonctionnalités proposées par un tel appareil de ventilation sont limitées soit à la simple ventilation soit au simple éclairage. Un autre objectif de la présente invention est donc de proposer un appareil de ventilation qui comprend des fonctionnalités combinées de ventilation et d'éclairage mais également des fonctionnalités additionnelles. Un autre inconvénient encore réside dans le fait que la ventilation et l'éclairage fonctionnent par des programmes indépendants ce qui ne facilite pas l'usage d'un tel appareil de ventilation car cela nécessite une mise en marche de l'éclairage d'un côté et de la ventilation de l'autre. Un autre objectif de la présente invention est donc de proposer un procédé d'utilisation d'un appareil de ventilation qui permette de mettre en marche dans le même temps et de manière dépendante l'ensemble des fonctionnalités de l'appareil de ventilation sans multiplier les interactions avec l'unité de contrôle.

Afin d'atteindre cet objectif, l'invention concerne un procédé de commande d'un système de ventilation selon la revendication 1.

Selon l'invention le procédé de commande du système de ventilation comprend en outre, entre l'étape de sélection du programme de fonctionnement et l'étape d'émission du signal de commande unique mentionnées précédemment, une étape de récupération des données de commande, effectuée par un processeur intégré dans l'unité de contrôle, qui consiste à récupérer dans une mémoire intégrée dans l'unité de contrôle les premières données de commande et au moins les secondes données de commande en fonction du programme de fonctionnement sélectionné, et une étape de compilation de ces données de commande, qui consiste à effectuer des opérations logiques sur ces données de commande afin de corréler ces données de commande et de les intégrer dans le signal de commande.

L'invention concerne également un système de ventilation selon la revendication 1, qui met en œuvre le procédé de commande énoncé précédemment.

Selon l'invention le dispositif lumineux du ventilateur est composé d'une ou plusieurs diodes électroluminescentes blanches qui forment un luminaire dont la puissance totale est comprise entre 3 Watts et 150 Watts.

Toujours selon l'invention le dispositif lumineux du ventilateur est composé d'une ou plusieurs diodes électroluminescentes de couleur qui forment un luminaire dont la puissance totale est comprise entre 3 Watts et 150 Watts. Selon l'invention le moteur du ventilateur est un moteur sans balais appelé moteur brushless et il est intégré dans un dispositif comprenant des pales de ventilation.

Selon une variante de réalisation de l'invention l'unité de contrôle du ventilateur est disposée à distance du ventilateur et comprend un dispositif de transmission et de réception sans fil des données de commande.

Selon l'invention le ventilateur comprend un dispositif de transmission et de réception sans fil des données de commande et une mémoire interne pour stocker ces données de commande.

Selon l'invention le dispositif de transmission et de réception sans fil des données de commande de l'unité de contrôle et le dispositif de transmission et de réception sans fil des données de commande du ventilateur communiquent entre eux par des signaux radio fréquence.

Selon l'invention le ventilateur peut communiquer avec un module distant connecté à internet.

Selon l'invention l'unité de contrôle peut communiquer avec un module distant connecté à internet.

Toujours selon l'invention la mémoire interne du ventilateur est programmable à distance.

D'autres caractéristiques et avantages de la présente invention apparaîtront plus clairement à la lecture de la description détaillée qui suit d'un mode de réalisation de l'invention donné à titre d'exemple nullement limitatif et illustré par les dessins annexés, dans lesquels :

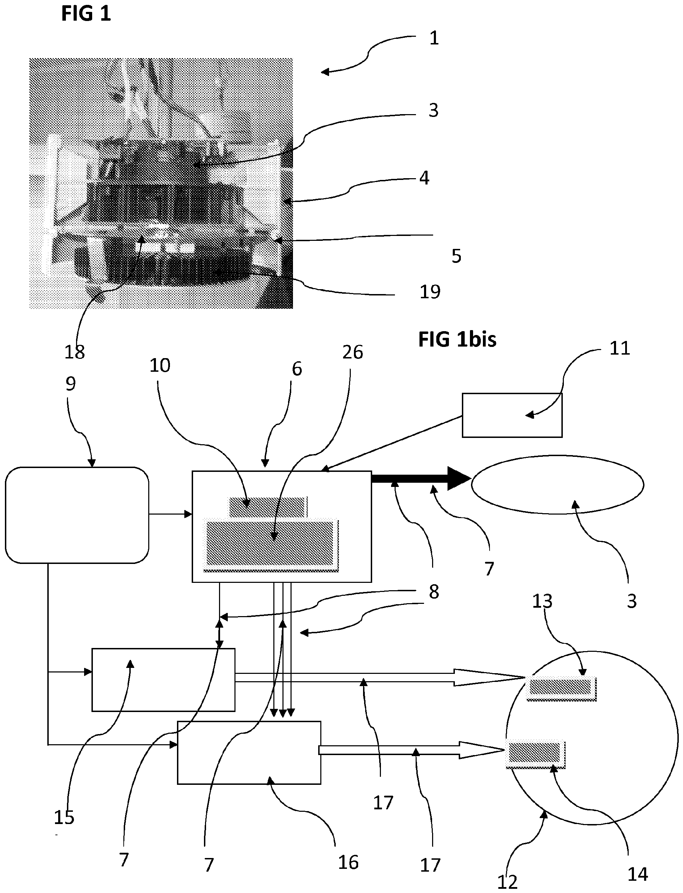

- la figure 1 est une vue en coupe d'un ventilateur conforme à l'invention ;
- la figure 1bis est un schéma synoptique de l'électronique du ventilateur conforme à l'invention ;
- la figure 2 est une vue en perspective d'un ventilateur conforme à l'invention ;
- la figure 3 est un schéma logique du procédé d'utilisation d'un ventilateur conforme à l'invention.

Comme on peut le voir sur les figures 1 ou 1bis, un ventilateur (1) conforme à l'invention est équipé d'un moteur (3) et le moteur (3) est intégré dans un dispositif (4) comprenant des pales (5) de ventilation. Dans un mode de réalisation préféré ce moteur (3) est un moteur (3) sans balais de type brushless.

Le ventilateur (1) comprend également une carte électronique (6) de gestion et de commande. Cette carte électronique (6) est reliée au moteur (3) et permet de commander au moins le moteur (3) en vitesse et en rotation.

Cette commande du moteur (3) se fait par modulation de largeur d'impulsions (7) et entre le moteur (3) et la carte électronique (6) de gestion on utilise une structure électronique de pont en H (8).

Le ventilateur (1) comprend une alimentation (9) sur secteur qui fonctionne de 120Veff à 230Veff et à des fréquences allant de 50Hz à 60Hz.

Le ventilateur (1) comprend également un dispositif (10) de réception sans fil de données. Ce dispositif (10) est relié à la carte électronique (6) de gestion et il peut être positionné soit dans la carte électronique (6) de gestion soit être déporté de celle-ci.

Le dispositif (10) de réception sans fil de données est adapté à recevoir des données du type radio fréquence et il peut également émettre des données. Un capteur de température (11) est relié à la carte électronique (6) de gestion. Le ventilateur (1) comprend également un dispositif lumineux (12). Ce dispositif lumineux (12) est composé d'une ou plusieurs diodes électroluminescentes blanches (13) qui forment un luminaire dont la puissance totale est comprise entre 3 Watts et 150 Watts. Ce dispositif lumineux (12) peut également comprendre une ou plusieurs diodes électroluminescentes de couleur (14) qui forment un luminaire dont la puissance totale est comprise entre 3 Watts et 150 Watts. Ces diodes électroluminescentes de couleur (14) sont du type RGB pour Red, Green, Blue et permettent d'obtenir l'ensemble du spectre de couleurs existantes.

Les diodes électroluminescentes blanches (13) et les diodes électroluminescentes de couleurs (14) peuvent être positionnées sur le même luminaire.

L'avantage d'utiliser des diodes électroluminescentes (13, 14) est que la consommation est plus faible qu'avec une ampoule classique. Le rendement est meilleur et donc réchauffement est plus faible.

Plus particulièrement, l'alimentation permettant d'alimenter les diodes électroluminescentes (13, 14) est une alimentation à découpage et cette alimentation se fait à courant constant.

Pour les diodes électroluminescentes blanches (13) on utilise une alimentation à découpage de 25W (15) et pour les diodes électroluminescentes de couleurs (14) type RGB, on utilise une alimentation à découpage de 10W (16).

La commande permettant de commander les diodes électroluminescentes (13, 14) est une commande à courant constant isolée (17).

Les alimentations à découpage (15, 16) apportent une robustesse aux perturbations du secteur telles que la foudre, les creux de tension, les parasites. L'immunité des diodes électroluminescentes (13, 14) vis-à-vis de ces perturbations est augmentée.

Les diodes électroluminescentes blanches (13) et les diodes électroluminescentes de couleur (14) sont positionnées dans le ventilateur (1) sur un pavé de support (18). A cause de l'élévation de la température due aux diodes électroluminescentes (13, 14), un radiateur (19) pour assurer un contact thermique est prévu à proximité du pavé de support (18). Ce contact permet de diminuer la chaleur du pavé de support (18).

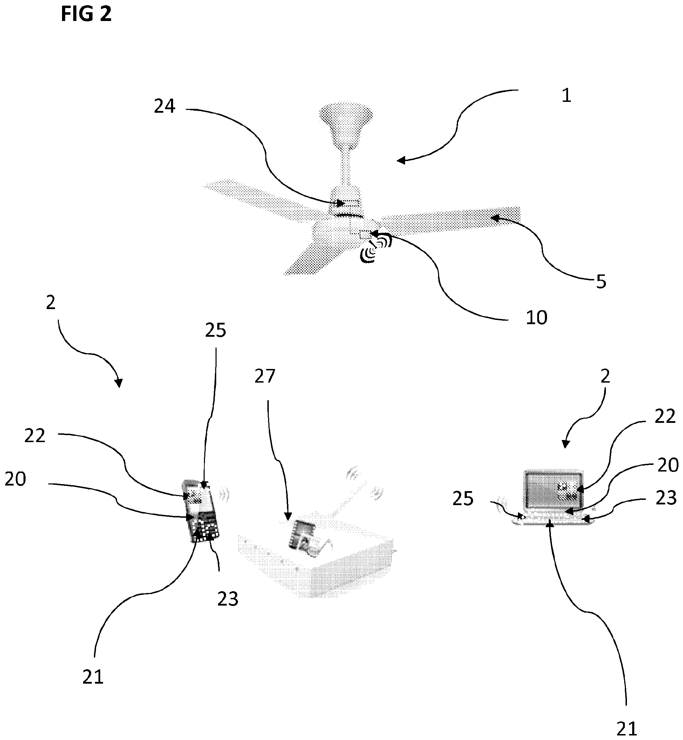

Comme cela est visible sur la figure 2, le ventilateur (1) comprend une unité (2) de contrôle. Cette unité (2) de contrôle comprend une interface (20) de sélection. Cette interface de sélection peut être un écran capacitif ou une dalle tactile.

L'unité (2) de contrôle possède des moyens (21) de mémorisation de façon à pouvoir garder en mémoire des programmes (22) de fonctionnement qui sont préenregistrés. Ces moyens (21) de mémorisation se déclinent sous forme de mémoire. Cette mémoire (21) peut être programmable.

L'interface (20) de sélection permet d'afficher ces programmes (22) de fonctionnement et un appui sur celle-ci permet de sélectionner un des programmes (22) de fonctionnement.

Les programmes (22) de fonctionnement comprennent au moins des premières données de commande pour le pilotage du moteur et au moins des secondes données de commande pour le pilotage d'au moins un dispositif secondaire (24) du ventilateur (1).

Outre le dispositif lumineux (12), le dispositif secondaire (24) du ventilateur (1) peut être un dispositif de pulvérisation de gaz ou de liquide, un dispositif sonore ou encore un dispositif vidéo.

Par dispositif de pulvérisation de gaz il faut entendre tous les dispositifs qui sont aptes à pulvériser des gaz tels que des parfums.

Ce dispositif de pulvérisation de gaz ou de liquide peut diffuser toutes sortes d'odeurs ou de parfums mais également il peut vaporiser des liquides comme de l'eau.

Le dispositif vidéo est un dispositif qui peut projeter des images ou des vidéos par l'intermédiaire d'un projecteur d'image ou de lumière.

L'unité (2) de contrôle comprend un processeur (23) qui peut accéder à la mémoire (21) et qui permet de récupérer les données de commande pour le pilotage du moteur (3) et des au moins secondes données de commande du dispositif secondaire (24).

Dans le cas où l'unité (2) de contrôle du ventilateur (1) est disposée à distance du ventilateur (1), celle-ci comprend un dispositif (25) de transmission sans fil des données de commande.

L'unité (2) de contrôle et le ventilateur (1) communiquent par des signaux du type radio fréquence.

La carte électronique (6) de gestion et de commande du ventilateur (1) comprend une mémoire interne (26) et les programmes (22) de fonctionnement peuvent être envoyés par l'unité (2) de contrôle au ventilateur (1) et stockés dans la mémoire interne (26) du ventilateur (1), toujours dans le cas où l'unité (2) de contrôle est disposée à distance du ventilateur (1).

Le ventilateur (1) ou l'unité (2) de contrôle peuvent également communiquer avec un module (27) distant connecté à internet.

De cette façon les programmes (22) de fonctionnement peuvent être récupérés d'internet par l'unité (2) de contrôle sur la mémoire (21) de l'unité (2) de contrôle ou la mémoire interne (26) du ventilateur.

L'unité (2) de contrôle peut être une télécommande avec un écran, une tablette, un Smartphone. Elle peut également être un ordinateur central ou une télévision.

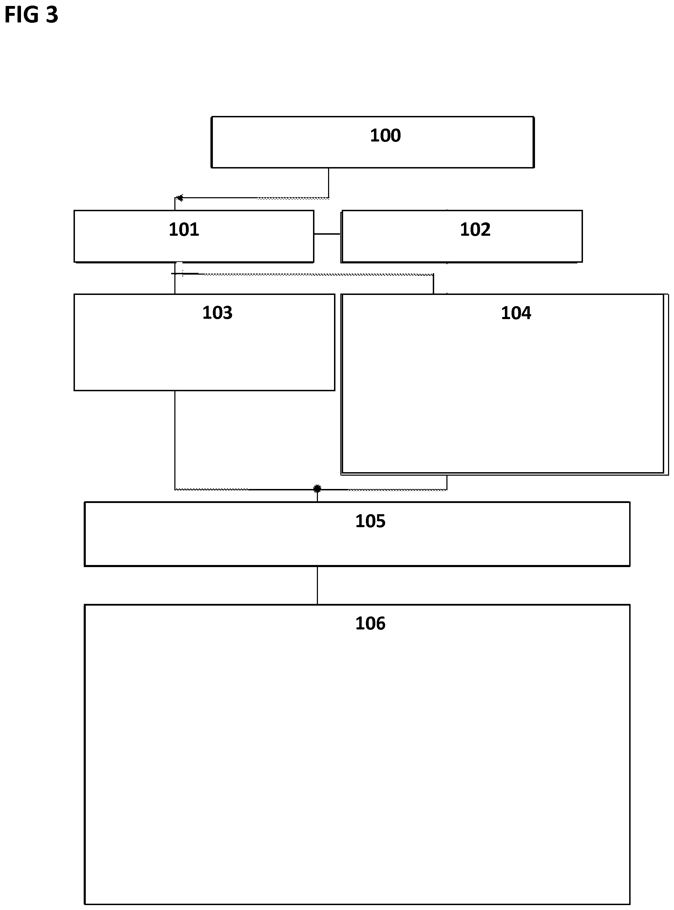

Comme on peut le voir sur la figure 3, le ventilateur (1) fonctionne de la manière suivante :
Le ventilateur (1) est mis sous tension dans une étape de démarrage (100).

Dans une étape (101) suivante l'unité (2) de contrôle est également mise sous tension et les programmes (22) de fonctionnement s'affichent sur l'interface (20) de celle-ci.

Au cours de cette étape (101) le ventilateur (1) communique avec l'unité (2) de contrôle et ils s'appairent.

Dans une autre étape (102) de fonctionnement, lors de l'allumage de l'unité (2) de contrôle, celle-ci s'appaire au module (27) distant directement connecté à internet. De cette manière l'unité (2) de contrôle peut récupérer des programmes (22) de fonctionnement sur internet.

Dans une étape suivante de sélection (103), un programme de fonctionnement préprogrammé est choisi sur l'unité (2) de contrôle.

Dans une variante, on peut procéder à une étape de programmation (104) du programme (22) de fonctionnement. On a ainsi un programme (22) de fonctionnement personnalisé dans lequel les données de commande sont rentrées manuellement sous forme de paramètres. Ainsi on peut trouver des paramètres comme la durée de fonctionnement, la date et l'heure d'activation, l'intensité lumineuse, la couleur du dispositif lumineux, la variation de lumière du dispositif lumineux, la vitesse de fonctionnement du dispositif de ventilation, la variation de ventilation, une consigne de température, des paramètres de diffusion d'odeurs ou de gaz, la projection d'une image, l'émission de sons.

Une fois les étapes de sélection (103) ou de programmation (104) du programme (22) de fonctionnement effectuées, les données de commande sont communiquées ou transmises au ventilateur (1) et sont stockées dans la mémoire interne (26) de celui-ci au cours d'une étape de transmission (105). Les données de commande sont transmises au ventilateur (1) sous la forme d'un signal de commande dans lequel les premières données de commande pour le pilotage du moteur (3) sont corrélées avec des secondes données de commande pour le pilotage du dispositif secondaire (24) du ventilateur (1). Par données corrélées, on entend que les premières données de commande ne sont pas indépendantes des secondes données de commande mais elles sont liées entre elles en ce sens qu'à une valeur particulière d'une première donnée de commande pour le pilotage du moteur sera associée une valeur particulière d'une seconde donnée de commande pour le pilotage du dispositif secondaire du ventilateur. Le signal de commande transportera donc en une seule fois ces deux informations qui sont liées.

Cette corrélation ou correspondance entre les premières et secondes données de commande est établie directement au sein du ou des programmes de fonctionnement préalablement sélectionnés. Dans ceux-ci sont préenregistrés sous forme de tables informatiques ou de bases de données des schémas de corrélation entre les données de commande.

Ces opérations seront effectuées par le processeur (23) intégré dans l'unité (2) de contrôle qui va compiler ces données de commande ce qui va consister à effectuer des opérations logiques sur ces données de commande afin d'en obtenir des données corrélées puis de les intégrer dans le signal de commande qui est alors unique dans le sens où un seul signal permet de transmettre l'ensemble des données de commande.

A titre d'exemple, à une vitesse particulière de rotation du moteur sera associée une couleur des diodes.

Ces programmes de fonctionnement comprennent également une information de temps. Cette information de temps correspond à une période qui délimite la durée pendant laquelle le signal de commande comprenant les premières et secondes données de commande sera transmis.

Comme dans l'exemple précédent, l'information de temps comprise dans les programmes permettra d'avoir une vitesse particulière de rotation du moteur corrélée avec une couleur particulière des diodes seulement pendant une période délimitée, puis à la fin de cette période soit le programme arrête d'envoyer le signal de commande soit il transmet un autre signal de commande avec une autre vitesse particulière de rotation du moteur corrélée avec une autre couleur particulière des diodes et ceci également pendant une autre période de temps qui peut être différente de la première et ainsi de suite.

Cette transmission des données au ventilateur se fait par des signaux radio fréquence.

Toujours dans cette étape de transmission (105), le ventilateur (1) peut recevoir une information du capteur de température (11).

Une fois l'ensemble des informations et données de commande reçues par la carte électronique (6) de gestion, on arrive à une étape de gestion (106) du ventilateur (1) où celui assure une gestion autonome de son fonctionnement en fonction des données de commande et des informations reçues.

Dans cette étape de gestion (106), le ventilateur (1) peut également envoyer des informations à l'unité (2) de contrôle.

On comprendra que diverses modifications et/ou améliorations évidentes pour l'homme du métier peuvent être apportées au mode de réalisation de l'invention décrit dans la présente description sans sortir du cadre de l'invention défini par les revendications annexées.

## Revendications

1. Procédé de commande d'un système de ventilation comprenant un ventilateur (1) et une unité (2) de contrôle **caractérisé en ce que** le procédé de commande comprend au moins les étapes suivantes :
- sélection d'un programme (22) de fonctionnement du ventilateur (1) parmi une pluralité de programmes de fonctionnement préenregistrés dans l'unité (2) de contrôle et les programmes de fonctionnement comprennent au moins des premières et des secondes données de commande du ventilateur ;
- et émission par l'unité (2) de contrôle d'un signal de commande comprenant au moins les premières données de commande pour le pilotage d'un moteur (3) qui sont corrélées avec au moins les secondes données de commande pour le pilotage d'au moins un dispositif secondaire (24) du ventilateur (1) choisi parmi :
- Un dispositif lumineux (12) ;
- Un dispositif de pulvérisation de gaz ou de liquide ;
- Un dispositif sonore ;
- Un dispositif vidéo.

2. Procédé de commande du système de ventilation selon la revendication précédente **caractérisé en ce que** le procédé de commande comprend en outre, entre l'étape de sélection du programme (22) de fonctionnement et l'étape d'émission du signal de commande unique mentionnées précédemment, une étape de récupération des données de commande, effectuée par un processeur (23) intégré dans l'unité (2) de contrôle, qui consiste à récupérer dans une mémoire (21) intégrée dans l'unité (2) de contrôle les premières données de commande et au moins les secondes données de commande en fonction du programme (22) de fonctionnement sélectionné, et une étape de compilation de ces données de commande qui consiste à effectuer des opérations logiques sur ces données de commande afin de corréler ces données de commande et de les intégrer dans le signal de commande.

3. Système de ventilation comprenant un ventilateur et une unité (2) de contrôle mettant en œuvre le procédé de commande selon l'une des revendications précédentes **caractérisé en ce que** :
- l'unité (2) de contrôle comprend une interface (20) de sélection d'un programme (22) de fonctionnement préenregistré dans l'unité (2) de contrôle ;
- l'unité (2) de contrôle comprend une mémoire (21) dans laquelle est stockée au moins des premières données de commande pour le pilotage d'un moteur (3) et au moins des secondes données de commande pour le pilotage d'au moins un dispositif secondaire (24) du ventilateur (1) et le dispositif secondaire (24) du ventilateur (1) est choisi parmi ;
∘ Un dispositif lumineux ;
∘ Un dispositif de pulvérisation de gaz ou de liquide ;
∘ Un dispositif sonore ;
∘ Un dispositif vidéo.
- l'unité (2) de contrôle comprend un processeur (23) qui peut accéder à la mémoire (21) et qui permet de récupérer les données de commande pour le pilotage du moteur (3) et des au moins secondes données de commande relatives au programme sélectionné et de compiler ces données de commande de manière à obtenir un signal de commande dans lequel les données sont corrélées.

4. Système de ventilation selon la revendication précédente **caractérisé en ce que** le dispositif lumineux (12) est composé d'une ou plusieurs diodes électroluminescentes blanches (13) qui forment un luminaire dont la puissance totale est comprise entre 3 Watts et 150 Watts.

5. Système de ventilation selon la revendication 3 ou la revendication 4 **caractérisé en ce que** le dispositif lumineux (12) est composé d'une ou plusieurs diodes électroluminescentes de couleur (14) qui forment un luminaire dont la puissance totale est comprise entre 3 Watts et 150 Watts.

6. Système de ventilation selon l'une des revendications 3 à 5 **caractérisé en ce que** le moteur est un moteur (3) sans balais appelé moteur brushless et il est intégré dans un dispositif (4) comprenant des pales (5) de ventilation.

7. Système de ventilation selon l'une des revendications 3 à 6 **caractérisé en ce que** l'unité (2) de contrôle est disposée à distance du ventilateur (1) et comprend un dispositif (25) de transmission et de réception sans fil des données de commande.

8. Système de ventilation selon la revendication précédente **caractérisé en ce que** le ventilateur (1) comprend un dispositif (10) de transmission et de réception sans fil des données de commande et une mémoire interne (26) pour stocker ces données de commande.

9. Système de ventilation selon la revendication précédente **caractérisé en ce que** le dispositif (25) de transmission et de réception sans fil des données de commande de l'unité (2) de contrôle et le dispositif (10) de transmission et de réception sans fil des données de commande du ventilateur (1) communiquent entre eux par des signaux radio fréquence.

10. Système de ventilation selon l'une des revendications 3 à 9 **caractérisé en ce que** le ventilateur (1) peut communiquer avec un module distant (27) connecté à internet.

11. Système de ventilation selon l'une des revendications 3 à 10 **caractérisé en ce que** l'unité (2) de contrôle peut communiquer avec un module distant (27) connecté à internet.

12. Système de ventilation selon les revendications 3 à 11 **caractérisé en ce que** la mémoire interne (26) du ventilateur (1) est programmable à distance.

## Patentansprüche

1. Verfahren zum Steuern eines Lüftungssystems, umfassend einen Lüfter (1) und eine Kontrolleinheit (2), **dadurch gekennzeichnet, dass** das Steuerverfahren mindestens die folgenden Schritte umfasst:
- Auswählen eines Betriebsprogramms (22) des Lüfters (1) unter mehreren von in der Kontrolleinheit (2) aufgezeichneten Betriebsprogrammen, und wobei die Betriebsprogramme mindestens erste und zweite Steuerdaten des Lüfters umfassen;
- und Senden eines Steuersignals durch die Kontrolleinheit (2), das mindestens die ersten Steuerdaten zum Lenken eines Motors (3) umfasst, die mit mindestens den zweiten Steuerdaten zum Lenken mindestens einer sekundären Vorrichtung (24) des Lüfters (1) korreliert sind, die ausgewählt ist aus:
- einer Leuchtvorrichtung (12);
- einer Vorrichtung zum Sprühen von Gas oder Flüssigkeit;
- einer akkustischen Vorrichtung;
- einer Videovorrichtung.

2. Verfahren zum Steuern eines Lüftungssystems gemäß dem vorhergehenden Anspruch, **dadurch gekennzeichnet, dass** das Verfahren zum Steuern ferner zwischen dem vorher erwähnten Schritt des Auswählens des Betriebsprogramms (22) und dem vorher erwähnten Schritt des Sendens des einzelnen Steuersignals einen Schritt des Abrufens der Steuerdaten, der von einem in der Kontrolleinheit (2) integrierten Prozessor (23) ausgeführt wird und der darin besteht, aus einem in der Kontrolleinheit (2) integrierten Speicher (21) die ersten Steuerdaten und mindestens die zweiten Steuerdaten gemäß dem ausgewählten Betriebsprogramm (22) abzurufen, und einen Schritt des Kompilierens dieser Steuerdaten umfasst, der darin besteht, logische Operationen an diesen Steuerdaten durchzuführen, um diese Steuerdaten zu korrelieren und sie in dem Steuersignal zu integrieren.

3. Lüftungssystem, umfassend einen Lüfter (1) und eine Kontrolleinheit (2), die das Verfahren zum Steuern gemäß einem der vorhergehenden Ansprüche umsetzt, **dadurch gekennzeichnet, dass**:
- eine Kontrolleinheit (2) eine Schnittstelle (20) zum Auswählen eines in der Kontrolleinheit (2) aufgezeichneten Betriebsprogramms (22) umfasst;
- die Kontrolleinheit (2) einen Speicher (21) umfasst, in dem mindestens erste Steuerdaten zum Lenken eines Motors (3) und mindestens zweite Steuerdaten zum Lenken mindestens einer sekundären Vorrichtung (24) des Lüfters (1) gespeichert sind, und wobei die sekundäre Vorrichtung (24) des Lüfters (1) ausgewählt ist aus:
∘ einer Leuchtvorrichtung;
∘ einer Vorrichtung zum Sprühen von Gas oder Flüssigkeit;
∘ einer akkustischen Vorrichtung;
∘ einer Videovorrichtung;
- die Kontrolleinheit (2) einen Prozessor (23) umfasst, der auf den Speicher (21) zugreifen kann und der es ermöglicht, die Steuerdaten zum Lenken des Motors (3) und mindestens zweite Steuerdaten bezüglich des ausgewählten Programms abzurufen und diese Steuerdaten zu kompilieren, um ein Steuersignal zu erhalten, in dem die Daten korreliert sind.

4. Lüftungssystem nach dem vorhergehenden Anspruch **dadurch gekennzeichnet, dass** die Leuchtvorrichtung (12) aus einer oder mehreren weißen Leuchtdioden (13) besteht, die eine Leuchte bilden, deren Gesamtleistung zwischen 3 Watt und 150 Watt liegt.

5. Lüftungssystem nach Anspruch 3 oder Anspruch 4, **dadurch gekennzeichnet, dass** die Leuchtvorrichtung (12) aus einer oder mehreren Farbleuchtdioden (14) besteht, die eine Leuchte bilden, deren Gesamtleistung zwischen 3 Watt und 150 Watt liegt.

6. Lüftungssystem nach einem der Ansprüche 3 bis 5, **dadurch gekennzeichnet, dass** der Motor ein bürstenloser Motor (3) ist, der als Brushless-Motor bezeichnet wird, und wobei er in einer Vorrichtung (4) integriert ist, die Lüftungsblätter (5) umfasst.

7. Lüftungssystem nach einem der Ansprüche 3 bis 6, **dadurch gekennzeichnet, dass** die Kontrolleinheit (2) in einem Abstand vom Lüfter (1) angeordnet ist und eine Vorrichtung (25) zum drahtlosen Übertragen und Empfangen von Steuerdaten umfasst.

8. Lüftungssystem gemäß dem vorhergehenden Anspruch, **dadurch gekennzeichnet, dass** der Lüfter (1) eine Vorrichtung (10) zum drahtlosen Übertragen und Empfangen von Steuerdaten und einen internen Speicher (26) zum Speichern dieser Steuerdaten umfasst.

9. Lüftungssystem gemäß dem vorhergehenden Anspruch, **dadurch gekennzeichnet, dass** die Vorrichtung (25) zum drahtlosen Übertragen und Empfangen der Steuerdaten der Kontrolleinheit (2) und die Vorrichtung (10) zum drahtlosen Übertragen und Empfangen der Steuerdaten des Lüfters (1) über Hochfrequenzsignale miteinander kommunizieren.

10. Lüftungssystem nach einem der Ansprüche 3 bis 9, **dadurch gekennzeichnet, dass** der Lüfter (1) mit einem mit dem Internet verbundenen Fernmodul (27) kommunizieren kann.

11. Lüftungssystem nach einem der Ansprüche 3 bis 10, **dadurch gekennzeichnet, dass** die Kontrolleinheit (2) mit einem mit dem Internet verbundenen Fernmodul (27) kommunizieren kann.

12. Lüftungssystem nach einem der Ansprüche 3 bis 11, **dadurch gekennzeichnet, dass** der interne Speicher (26) des Lüfters (1) fernprogrammierbar ist.

## Claims

1. Method for controlling a ventilation system comprising a fan (1) and a control unit (2) **characterised in that** the control method comprises at least the following steps:
- selecting an operating program (22) for the operation of the fan (1) from among a plurality of operating programs pre-recorded in the control unit (2), and the operating programs comprise at least a first and second control data for the fan;
- and transmitting by the control unit (2) of a control signal including at least the first control data to control a motor (3), which are correlated with at least the second control data to control at least one secondary device (24) of the fan (1) selected from among:
- A lighting device;
- A gas or liquid spraying device;
- An acoustic device
- A video device.

2. Method for controlling the ventilation system according to the preceding claim **characterised in that** the control method further comprises, between the aforementioned operating program selection step (22) and single control signal transmission step, a control data retrieval step, performed by a processor (23) integrated into the control unit (2), which consists of retrieving in a memory (21) integrated into the control unit (2), the first control data and, at least, the second control data based on the operating program (22) selected, and a step of compiling these control data, which consists of performing logic operations on these control data in order to correlate the control data and integrate the control data into the control signal.

3. Ventilation system comprising a fan (1) and a control unit (2) implementing the control method according to one of the preceding claims **characterised in that**:
- the control unit (2) comprises an interface (20) to select an operating program (22) pre-recorded in the control unit (2);
- the control unit (2) has a memory (21) wherein are stored at least the first control data to pilot a motor (3) and at least the second control data to pilot at least one secondary device (24) of the fan (1), and the secondary device (24) of the fan (1) is selected from among;
∘ A lighting device;
∘ A gas or liquid spraying device;
∘ An acoustic device
∘ A video device.
- the control unit (2) comprises a processor (23) which can access the memory (21) and which makes it possible to retrieve the control data to pilot the motor (3) and at least the second control data related to the program selected and to compile the control data to obtain a control signal in which the data are correlated.

4. Ventilation system according to the preceding claim **characterised in that** the lighting device (12) is comprised of one or more white light-emitting diodes (13) which form a luminaire of which the total power is comprised between 3 Watts and 150 Watts.

5. Ventilation system according to claim 3 or claim 4 **characterised in that** the lighting device (12) is comprised of one or more colour light-emitting diodes (14) which form a luminaire of which the total power is comprised between 3 Watts and 150 Watts.

6. Ventilation system according to one of claims 3 to 5 **characterised in that** the motor is a brushless motor (3) and is integrated into a device (4) comprising fan blades (5).

7. Ventilation system according to one of claims 3 to 6 **characterised in that** the control unit (2) is located away from the fan (1) and comprises a wireless control data transmission and receiving device (25).

8. Ventilation system according to the preceding claim **characterised in that** the fan (1) comprises a wireless control data transmission and receiving device (10) and an internal memory (26) to store this control data.

9. Ventilation system according to the preceding claim **characterised in that** the wireless control data transmission and receiving device (25) of the control unit (2) and the wireless control data transmission and receiving device (10) of the fan (1) communicate with each other using radio frequency signals.

10. Ventilation system according to one of claims 3 to 9 **characterised in that** the fan (1) can communicate with a remote module (27) connected to the internet.

11. Ventilation system according to one of claims 3 to 10 **characterised in that** the control unit (2) can communicate with a remote module (27) connected to the internet.

12. Ventilation system according to claims 3 to 11 **characterised in that** the internal memory (26) of the fan (1) can be programmed remotely.
